# FASCICULE DE BREVET EUROPEEN

(11) **EP 0 349 451 B1**
(45) Date de publication et mention de la délivrance du brevet: **03.03.1993**
(21) Numéro de dépôt: 89440058.9
(22) Date de dépôt: 21.06.1989
(51) Int. Cl.: C07K 7/10, A61K 37/02

(54) **Molécules peptidiques à action sur les germes à Gram positif**
Peptid-Moleküle, wirksam gegen Gram-positive Keime
Peptide molecules active against Gram-positive germs

(30) Priorité: 24.06.1988 FR 8808892
(43) Date de publication de la demande: 03.01.1990
(73) Titulaire: CENTRE NATIONAL DE LA RECHERCHE SCIENTIFIQUE, ETABLISSEMENT PUBLIC NATIONAL A CARACTERE SCIENTIFIQUE ET TECHNOLOGIQUE( CNRS), 75700 Paris (FR)
(72) Inventeur: Hoffmann, Jules Lab. de Biologie Générale, F-67000 Strasbourg (FR); Lambert, Jean Lab. de Biologie Générale, F-67000 Strasbourg (FR); Dimarcq, Jean-Luc Lab. de Biologie Générale, F-67000 Strasbourg (FR); Keppi, Elizabeth Lab. de Biologie Générale, F-67000 Strasbourg (FR); Reichhart, Jean-Marc Lab. de Biologie Générale, F-67000 Strasbourg (FR); Hoffmann, Danièle Lab. de Biologie Générale, F-67000 Strasbourg (FR); Fothergill, John, Marischal College Aberdeen AB9 1AS (GB); Van Dorselaer, Alain Lab. de Chimie Organique, 5, rue Blaise Pascal 67000 Strasbourg (FR); Luu, Bang Lab. de Chimie Organique, 5, rue Blaise Pascal 67000 Strasbourg (FR)
(74) Mandataire: Nuss, Pierre

(56) Documents cités:
- COMPTES RENDUS DE L'ACADEMIE DES SCIENCES PARIS, vol. 303, no. 5, 15 juillet 1986, "Série III SIENCES DE LA VIE", pages 155-160, Académie des Sciences: E. KEPPI et al.: "Immunologie. Recherches sur les mécanismes de défense antibactérienne chez les insectes: isolement de peptides antibactériens dans l'hémolymphe du Diptère Phormia terranovae"
- BIOLOGICAL ABSTRACTS, vol. 81, no. 11, 1986, page AB-554, résumé no. 102712, Biological Abstracts, Inc., Philadelphia, PA, US; E. KEPPI et al.: "Induced antibacterial proteins in the hemolymph of Phormia terranovae (Diptera): Purification and possible origin of the one protein", & INSECT BIOCHEM 16(2): 395-402 1986

## Description

La présente invention concerne les domaines de la médecine humaine et vétérinaire, phytosanitaire et de l'industrie alimentaire et a pour objet des molécules peptidiques à action sur les germes à Gram positif.

Il est connu, depuis longtemps, que les insectes sont capables de réactions immunitaires très efficaces. A titre d'exemple, il est possible de réaliser toutes sortes d'interventions chirurgicales sur des insectes sans prendre les moindres précautions d'asepsie, ces opérations n'entraînant jamais de septicémie. Ceci est dû au fait que la réponse immunitaire des insectes fait intervenir, dans un délai court, à savoir de l'ordre de quatre à six heures, la production de plusieurs peptides antibactériens présentant généralement un large spectre d'action.

Ainsi, il a été isolé et caractérisé, chez des Lépidoptères, deux familles de peptides antibactériens induits au cours de la réponse immunitaire, à savoir les cécropines et les attacines.

Un travail sur un autre groupe d'insectes, les Diptères, a permis d'isoler et de caractériser trois molécules, très voisines entre elles et très différentes des cécropines, ces molécules étant des antibiotiques tout à fait nouveaux qui sont synthétisées par lesdits insectes dans les heures suivant une blessure. Ces trois molécules sont connues sous la dénomination diptéricine A, B et C et sont des peptides antibactériens basiques de 9 K dont le spectre d'action est large au sein des bactéries à Gram négatif (à titre d'exemple, la masse moléculaire de la diptéricine A est de 8621 Da). Les résultats de ce travail ont été publiés notamment dans European Journal of Biochemistry vol. 171, pages 17 à 22, (1988). En effet, dans cette publication, il est traité de la synthèse de molécules à partir des larves du diptère Phormia terranovae. Ces études ont également fait l'objet de publications dans les COMPTES RENDUS DE L'ACADEMIE DES SCIENCES PARIS, vol. 303, no. 5, 15 juillet 1986, "Série III SCIENCES DE LA VIE", pages 155-160, Académie des Sciences ; E. KEPPI et al. : "IMMUNOLOGIE - Recherches sur les mécanismes de défense antibactérienne chez les insectes ; isolement de peptides antibactériens dans l'hémolymphe du Diptère Phormia terranovae" - et au document BIOLOGICAL ABSTRACTS, vol. 81, no. 11, 1986, page AB-554, no. 102712, Phildelphia, PA., US ; E. KEPPI et al. : "Induced antibacterial proteins in the hemolymph of Phormia terranovae (Diptera) : Purification and possible origin of the one protein" & INSECT BIOCHEM 16(2) ; 395-402 1986.

Cependant, ces nouvelles molécules, à l'état purifié, ne sont pas ou sont seulement faiblement actives sur les germes à Gram positif. Les diptéricines tuent les bactéries en phase exponentielle de croissance et le résultat de leur action est une lyse cellulaire.

En effet, on connaît, d'après ces documents, une analyse en acides aminés faisant état d'une séquence peptidique sans cystéine, et les molécules obtenues présentent une masse moléculaire de l'ordre de 9000 Da. Ces molécules peptidiques sont uniquement actives contre les bactéries Gram négatif.

La présente invention a pour objet des molécules peptidiques à action sur les germes à Gram positif.

Conformément à l'invention, ces molécules présentent la définition suivante :
peptides basiques de 40 acides aminés comportant 6 cystéines engagées dans 3 ponts disulfure, susceptibles d'être obtenues à partir de larves de l'insecte Phormia terranovae après immunisation, ces molécules ayant une activité contre des germes à Gram positif.

L'invention sera mieux comprise grâce à la description ci-après, qui se rapporte à un mode de réalisation préféré, donné à titre d'exemple non limitatif, et expliqué avec référence aux dessins schématiques annexés, dans lesquels :
la figure 1 est un diagramme représentant la séparation par chromatographie échangeuse de cations des protéines à activité anti-Micrococcus luteus, synthétisées par des larves de Phormia terranovae immunisées ;
les figures 2 et 3 sont des diagrammes représentant la purification de deux molécules peptidiques par chromatographie liquide à haute performance en phase inverse, et
la figure 4 représente un spectre antibactérien d'une molécule conforme à l'invention.

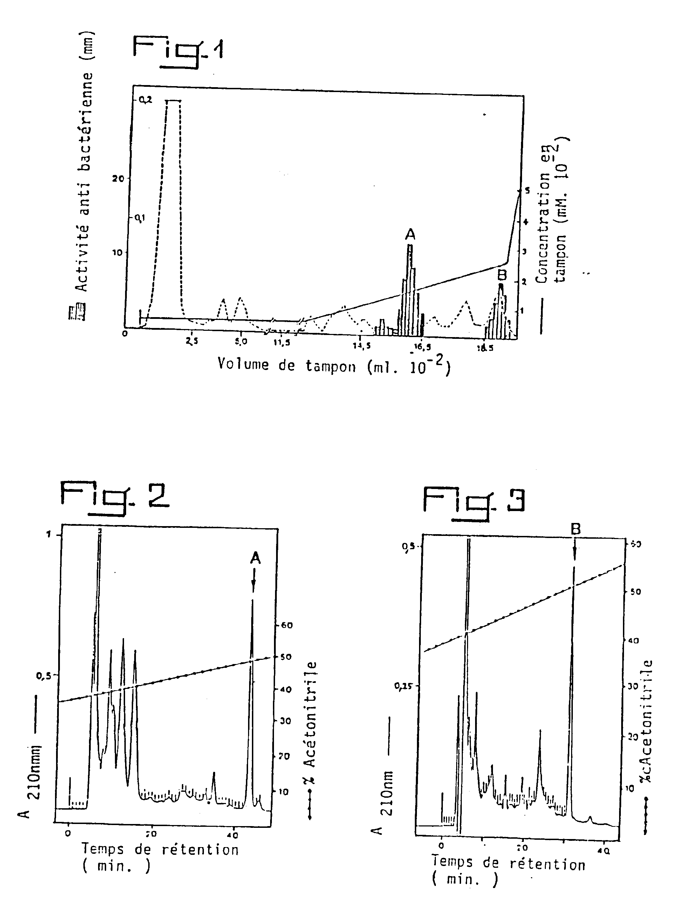

L'isolement et la caractérisation des nouvelles molécules peptidiques conformes à l'invention s'effectuent de la manière suivante :
Des larves de troisième stade du Diptère Phormia terranovae sont blessées par piqûre à l'aide d'une aiguille préalablement contaminée dans une culture bactérienne, par exemple Enterobacter cloacae en phase exponentielle de croissance, afin qu'elles entament leur processus d'immunisation. Au bout de 24 heures, du sang est prélevé par incision de la cuticule et les cellules sanguines sont enlevées par centrifugation à 36000 g pendant 20 minutes à 4°C. Le surnageant obtenu est chauffé, ensuite, pendant 4 minutes à 100°C, après addition d'acide acétique. Après centrifugation des protéines thermolabiles précipitées, le surnageant est appliqué sur une colonne de chromatographie échangeuse de cations équilibrée avec un tampon d'acétate d'ammonium de 40 mM et de pH 6,8. Après rinçage de la colonne avec le même tampon, les protéines fixées sont éluées par un gradient linéaire d'une solution d'acétate d'ammonium de molarité comprise entre 40mM et 500mM. Par cette technique connue, il est possible d'isoler des peptides actifs contre Micrococcus luteus par test d'étalement sur agar ou sur gélose ensemencés de germes bactériens. La figure 1 des dessins annexés représente le diagramme de séparation correspondant, dans lequel l'activité antibactérienne en millimètres de diamètre d'étalement est indiquée sur l'ordonnée de gauche, la concentration en tampons, en mm . 10⁻ ², sur l'ordonnée de droite et le volume de tampons, en ml . 10⁻ ² en abscisse.

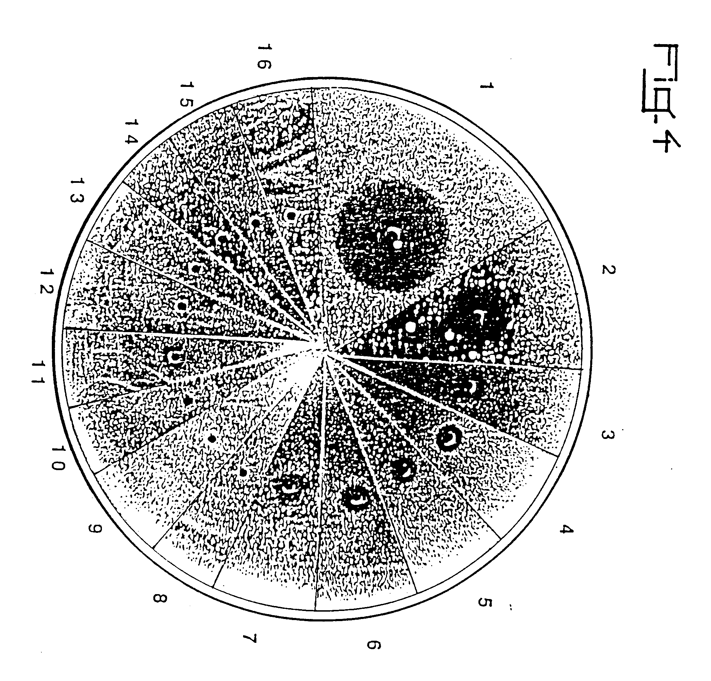

Ensuite, les molécules obtenues sont filtrées sur cartouche connue sous la dénomination commerciale Sep Pack C18 Waters et purifiées en chromatographie liquide à haute performance en phase inverse, couramment appelée HPLC phase inverse sur une colonne connue du type Baker-Bond C18 WP avec élution par gradient linéaire d'acétonitrile. Les figures 2 et 3 des dessins annexés représentent l'évolution des diagrammes de purification de deux molécules conformes à l'invention en fonction du temps de rétention indiqué en abscisse et du taux d'acétonitrile.

Le procédé décrit ci-dessus permet l'obtention, sous forme pure, de protéines anti-Micrococcus luteus représentées par les pics A et B aux figures 1 à 3.

Conformément à une caractéristique de l'invention, la molécule présente la séquence peptidique suivante :

1          10                    20                    30                    40
ATCDLLSGTG   INHSACAAHC   LLRGNRGGYC   NGKGVCVCRN          (A)

Selon une autre caractéristique de l'invention, la molécule présente la séquence peptidique suivante :

1          10                20                30                40
ATCDLLSGTG  INHSACAAHC  LLRGNRGGYC  NRKGVCVCRN          (B)

Les séquences peptidiques représentées ci-dessus se caractérisent par la présence de six cystéines en position homologue, tous les résidus étant identiques à l'exception du résidu 32, qui est une glycine dans la première molécule et une arginine dans la deuxième molécule. Ce remplacement explique le comportement différentiel des molécules précitées (A) et (B) en chromatographie échangeuse de cations, la dernière molécule (B) citée présentant un résidu, l'arginine, ayant plus de charges positives. Les pics A et B de la figure 1 des dessins annexés correspondent respectivement à la première et à la seconde molécules précitées.

Une mesure de la masse chimique des molécules précitées (A) et (B) a été réalisée par spectrométrie de masse en mode à bombardement par atome rapide positif en utilisant une résolution suffisamment basse, R étant égal à 1000, afin que le massif pseudo-moléculaire protoné apparaîsse comme un pic homogène. Dans ces conditions, la masse moléculaire mesurée pour la molécule (A) s'est établie à 4060,22 Da pour une masse attendue (calculée) de 4060,21 Da et celle mesurée pour la molécule (B) s'est établie à 4159,16 Da pour une masse attendue de 4159,35 Da. Cette mesure correspond à la forme oxydée des six cystéines, à savoir comporte six unités de masse en moins par rapport à la structure moléculaire calculée à partir de la structure primaire établie, prouvant que trois ponts cystéines sont formés. Une mesure de masse mono-isotopique, pour la molécule (A) a permis d'établir une masse de 4057,90 Da pour une masse attendue de 4057,81 Da.

L'activité des molécules peptidiques conformes à l'invention a été testée sur différentes souches bactériennes par un test d'ensemencement sur agar ou gélose, A cet effet, un spectre antibactérien de la molécule (A) a été réalisé à raison de 800 ng par essai sur les germes à Gram positif suivants :
1. Micrococcus luteus, et
2. Bacillus megaterium
3. à 7. Bacillus subtilis, suivant souches S3, Mo201, 6633, QB122 et QB935,
8. à 10. Bacillus thuringiensis, suivant souche Sm ^{r}, Rif ^{r} et 53137, et
11. Staphylococcus aureus, ainsi que sur
12. à 14. Escherichia coli, suivant souche D 31, BZB1011 et 7624,
15. Enterobacter cloacae (souche β 12) et
16. Pseudomonas aeruginosa.

Le spectre antibactérien obtenu, représenté à la figure 4 des dessins annexés, a permis de constater que les molécules conformes à l'invention sont actives sur les germes à Gram positif : Micrococcus luteus et Bacillus megaterium, de manière très efficace et, avec une efficacité un peu moindre sur les autres germes à Gram positif, à savoir Bacillus subtilis, Bacillus thuringiensis et Staphylococcus aureus, alors qu'elles ne sont pas actives sur les germes à Gram négatif : Escherichia coli, Enterobacter cloacae et Pseudomonas aeruginosa.

Les nouvelles molécules peptidiques conformes à l'invention peuvent avantageusement être utilisées pour l'obtention d'un médicament. Un tel médicament peut avantageusement servir comme agent antibactérien.

Selon une autre caractéristique de l'invention, les nouvelles molécules peptidiques ont une action bactéricide sur les germes à Gram positif. Cette caractéristique a été démontrée par une série d'expériences in vitro. La cible principale des molécules conformes à l'invention est la membrane cytoplasmique des bactéries. Ces molécules sont bactéricides mais ne sont pas bactériolytiques.

Les molécules conformes à l'invention, qui pourraient avantageusement être appelées phormicines (du nom de l'insecte Phormia terranovae), sont de nouveaux antibiotiques de taille relativement petite à spectre d'action spécifique pour les germes à Gram positif. Ces molécules sont plus particulièrement applicables dans les domaines de l'industrie alimentaire, phytosanitaire et de la médecine humaine et vétérinaire. Leur utilisation dans le domaine agro-alimentaire, en particulier comme peptides antibactériens inductibles d'insectes est particulièrement intéressante.

Les gènes des molécules peptidiques conformes à l'invention peuvent également être clonés et les gènes (ou les ADN complémentaires) peuvent être introduits dans plusieurs systèmes d'expression tels que la levure ou le baculovirus, ou être produites en masse en fermentateurs. Ces molécules peuvent également être utilisées dans des procédés de mutagénèse afin d'isoler des mutants ayant de nouvelles propriétés antibactériennes. De même, les gènes codant pour ces molécules peuvent être introduits dans des plantes afin d'augmenter leur résistance à des agents pathogènes.

Les molécules peptidiques selon l'invention constituent, en outre, les compléments indispensables d'une stratégie biotechnologique basée sur l'utilisation des cécropines et des diptéricines qui agissent sur des germes à Gram négatif. Par ailleurs, leur faible taille et leur résistance au traitement à la chaleur et aux conditions acides présentent également un aspect très favorable pour une utilisation biotechnologique. De plus, ces nouvelles molécules peuvent agir, dans le domaine de l'industrie alimentaire, comme agent empêchant la contamination par des germes à Gram positif, pendant la fabrication et, après la fabrication, comme agent conservateur. Dans le domaine phytosanitaire, ces molécules peuvent être utilisées en lieu et place des produits chimiques usuels.

Dans le cas de sélection d'analogues, après mutagénèse du gène, pour une adaptation à des besoins spécifiques des industries agro-alimentaires et pharmaceutiques, ces analogues peuvent être intégrés dans des souches de levure ou de bactéries lactiques couramment utilisées en production, afin d'éliminer les contaminations bactériennes nocives parfois présentes dans ces produits.

Dans le domaine phytosanitaire, les gènes correspondant aux mutants donnant des peptides efficaces, peuvent éventuellement être intégrés dans le génome de plantes choisies afin de les rendre plus résistantes à des maladies d'origine bactérienne.

Bien entendu, la présente invention ne se limite nullement aux indications et aux applications décrites. D'autres indications et applications sont possibles, ainsi que des modifications aux indications et aux applications décrites, sans sortir pour autant du domaine de protection de l'invention.

## Revendications

1. Molécules peptidiques caractérisées en ce qu'elles présentent la définition suivante :
peptides basiques de 40 acides aminés comportant 6 cystéines engagées dans 3 ponts disulfure, susceptibles d'être obtenus à partir de larves de l'insecte Phormia terranovae après immunisation, ces molécules ayant une activité contre des germes à Gram positif.

2. Molécule suivant la revendication 1, caractérisée en ce qu'elle présente la séquence peptidique suivante :

1 10 20 30 40
ATCDLLSGTG INHSACAAHC LLRGNRGGYC NGKGVCVCRN (A)

3. Molécule suivant la revendication 1, caractérisée en ce qu'elle présente la séquence peptidique suivante :

1          10          20          30          40
ATCDLLSGTG  INHSACAAHC  LLRGNRGGYC  NRKGVCVCRN  (B)

4. Utilisation de molécules suivant l'une quelconque des revendications 1 à 3 pour l'obtention d'un médicament.

5. Utilisation de molécules suivant l'une quelconque des revendications 1 à 3 pour l'obtention d'un produit de traitement phytosanitaire.

6. Molécules, suivant l'une quelconque des revendications 1 à 3, pour utilisation comme agent antibactérien.

7. Molécules, suivant l'une quelconque des revendications 1 à 3, pour utilisation comme agent antibactérien sur les germes à Gram positif.

8. Molécules, suivant l'une quelconque des revendications 1 à 3, pour utilisation comme agent bactéricide.

9. Molécules, suivant l'une quelconque des revendications 1 à 3, pour utilisation comme agent antibactérien sur les germes à Gram positif dans le domaine de l'industrie alimentaire.

## Patentansprüche

1. Peptidmoleküle, gekennzeichnet durch folgende Beschreibung: basische Peptide von 40 Aminosäuren, beinhaltend 6 in drei Disulfidbrücken gebundene Cysteine, die aus Larven des Insekts Phormia terranovae nach Immunisierung gewonnen werden können, wobei diese Moleküle gegen Gram-positive Keime wirksam sind.

2. Moleküle nach Anspruch 1, dadurch gekennzeichnet, daß es folgende Peptidreihe aufweist:

1          10          20          30          40

ATCDLLSGTG    INHSACAAHC    LLRGNRGGYC    NGKGVCVCRN    (A)

3. Moleküle nach Anspruch 1, dadurch gekennzeichnet, daß es folgende Peptidreihe aufweist:

1          10          20          30          40

ATCDLLSGTG  INHSACAAHC  LLRGNRGGYC  NRKGVCVCRN  (B)

4. Verwendung der Moleküle nach einem der Ansprüche 1 bis 3 zur Gewinnung eines Medikaments.

5. Verwendung der Moleküle nach einem der Ansprüche 1 bis 3 zur Gewinnung eines pflanzlichen Heilmittels.

6. Moleküle nach einem der Ansprüche 1 bis 3 zur Verwendung als antibakterielles Mittel.

7. Molekül nach einem der Ansprüche 1 bis 3 zur Verwendung als antibakterielles Mittel gegen Gram-positive Keime.

8. Molekül nach einem der Ansprüche 1 bis 3 zur Verwendung als keimtötendes Mittel.

9. Molekül nach einem der Ansprüche 1 bis 3 zur Verwendung als antibakterielles Mittel gegen die Gram-positiven Keime im Bereich der Lebensmittelindustrie.

## Claims

1. Peptide molecules, characterised in that they have the following definition:
basic peptides of 40 amino-acids containing 6 cysteines bound in 3 disulphide bridges obtainable from larvae of the insect Phormia terranovae after immunisation, these molecules being active against Gram-positive bacteria.

2. Molecule according to claim 1, characterised in that it has the following peptide sequence:

1          10                  20                  30                  40
ATCDLLSGTG   INHSACAAHC   LLRGNRGGYC   NGKGVCVCRN                    (A)

3. Molecule according to claim 1, characterised in that it has the following peptide sequence:

1          10          20          30          40
ATCDLLSGTG  INHSACAAHC  LLRGNRGGYC  NRKGVCVCRN          (B)

4. Use of molecules according to any one of claims 1 to 3 for obtaining a drug.

5. Use of molecules according to any one of claims 1 to 3 for obtaining a product for plant-protecting treatment.

6. Molecules according to any one of claims 1 to 3 for use as an anti-bacterial agent.

7. Molecules according to any one of claims 1 to 3 for use as an anti-bacterial agent on Gram-positive bacteria.

8. Molecules according to any one of claims 1 to 3 for use as a bactericidal agent.

9. Molecules according to any one of claims 1 to 3 for use as an anti-bacterial agent on Gram-positive bacteria in the food industry.
